(19) Europäisches Patentamt
European Patent Office
Office européen des brevets

(11) Publication number: **0 198 034 B1**

(12) **EUROPEAN PATENT SPECIFICATION published in accordance with Art. 158(3) EPC**

(45) Date of publication of patent specification: **12.06.91**

(51) Int. Cl.[5]: **A61K 9/70**, A61L 15/16

(21) Application number: **85905267.2**

(22) Date of filing: **09.10.85**

(86) International application number: **PCT/US85/01951**

(87) International publication number: **WO 86/02272 (24.04.86 86/09)**

# (54) PHYSIOLOGICAL MEANS OF ENHANCING TRANSDERMAL DELIVERY OF DRUGS.

(30) Priority: **11.10.84 US 659919**

(43) Date of publication of application: **22.10.86 Bulletin 86/43**

(45) Publication of the grant of the patent: **12.06.91 Bulletin 91/24**

(84) Designated Contracting States: **AT BE CH DE FR GB IT LI LU NL SE**

(56) References cited:
**EP-A- 0 052 074**
**GB-A- 2 093 694**
**US-A- 376 886**
**US-A- 4 031 894**

**See also references of WO8602272**

(73) Proprietor: **Key Pharmaceuticals, Inc.**
**2000 Galloping Hills Road**
**Kenilworth New Jersey 07033(US)**

(72) Inventor: **BODOR, Nicholas**
**7211 S.W. 97th Lane**
**Gainesville, FL 32608(US)**

(74) Representative: **Werner, Hans-Karsten, Dr. et al**
**Deichmannhaus am Hauptbahnhof**
**W-5000 Köln 1(DE)**

**EP 0 198 034 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the field of transdermal delivery systems and to methods of enhancing the transdermal delivery of pharmaceutically active drugs from such systems. More specifically, the invention relates to such systems which include an anticholinergic agent in an amount sufficient to have a local antisecretory effect on skin, such an amount being insufficient to cause a systemic effect. The invention also relates to pre-treating the skin with anticholinergic agents so as to locally inhibit secretion from the skin and thereby eliminate the formation of an aqueous layer between any delivery system and the skin so as to enhance penetration of drugs from transdermal delivery systems.

It is well known that many drugs if taken orally, are destroyed on the first pass through the liver. It is also well known that when many drugs are taken orally, their rate of absorption into the body is not constant. In view of such difficulties, a number of different drug delivery systems have been developed. Recently, the use of transdermal delivery systems have met with increasing interest by researchers in the pharmaceutical drug delivery field.

U.S. Patent 4,291,015 to Keith, et al. discloses the use of a polymeric diffusion matrix for the sustained release of pharmaceutically active drugs. The matrix is covered by a backing layer and applied to the skin where diffusion of the pharmaceutically active drug occurs and the drug is transdermally delivered to the patient. Although U.S. Patent 4,291,015 discloses transdermal delivery of nitroglycerin, other drugs may be delivered by utilizing the same or a similar matrix, as disclosed in U.S. Patent 4,292,820; 4,292,302; and 4,292,303.

U.S. Patent 4,409,206 discloses the use of a different type of transdermal delivery system whereby the pharmaceutically active drug is dispersed within an adhesive (see also U.S. Patent 4,390,520). In accordance with such systems, the pharmaceutically active drug is dispersed in a pressure-sensitive adhesive which is adhered to the skin. The drug then diffuses from the adhesive through the skin for delivery to the patient. EP-A-0 052 074 describes the use of an effective amount of an anticholinergic agent in the form of parenteral applicable systems hindering the secretion of gastric acid, wherein the anticholinergic agent is applicated transdermal, intradermal, subcutaneous or intramuscular.

Other transdermal systems involve the use of a matrix which is in diffusional contact with a reservoir which contains the pharmaceutically active drug. The drug diffuses to the matrix and then to the skin and eventually to the patient. Still other compositions include an active ingredient in a salve or ointment which is applied to skin. The active ingredient diffuses from the salve to the skin and enters the body transdermally.

Each of the systems have various advantages and disadvantages with respect to the transdermally delivery of pharmaceutically active drugs. Certain systems may be useful in connection with one type of pharmaceutically active drug and not useful in connection with another. The ability to include one pharmaceutically active drug within a given system depends on various factors such as the compatibility of the drug with the system and the effects of the drugs on the system such as its ability to dissolve in the system.

As indicated above, the systems have various advantages, disadvantages, and degrees of usefulness in connection with different types of drugs. Without experimentation, it is offen impossible to accurately predict the usefulness of any particular system with any particular pharmaceutically active drug. However, the present inventor has found that all the systems do have certain similarities and more importantly a common undesirable feature. More specifically, they all operate by transporting the pharmaceutically active drug to the skin for transdermal delivery. In order to accomplish this, the system must be placed in intimate contact with the skin. Since normal skin will perspire such will create an aqueous layer between the skin and the system. It is this aqueous layer which causes the common disadvantage.

Perspiration may well be increased beneath a transdermal delivery system and any perspiration beneath a delivery system causes an outflow of water and other water soluble body salts from the skin. This increased outflow increases the size of the aqueous layer and thus hinders the desired inflow of the pharmaceutically active drug through the skin. When the system remains in place for a substantial period of time (24 hours is common), the outflow of perspiration can build up a substantial aqueous layer between the delivery system and the skin. This impediment is especially great if the pharmaceutically active drug is insoluble with respect to the aqueous layer or if the drug is in any way incompatible with the aqueous layer or coagulates upon contact with it. The present invention is directed to elimination of the undesirable outflow of perspiration from the skin and the undesirable accumulation of that perspiration (aqueous layer formation) between the skin and the drug delivery system.

## SUMMARY OF THE INVENTION

It is a primary object of the present invention to provide a physiological means of enhancing transdermal delivery of drugs.

Therefore, this invention relates to a transdermal delivery system capable of providing a desirable flow interface between the delivery system and skin surface comprising:

a pharmaceutically active drug;

a dermatologically and pharmacologically acceptable carrier means for the drug; and

an anticholinergic agent in an amount sufficient to have a local antisecretory effect, such an amount being insufficient to cause a systemic effect and

a transdermal delivery system,

wherein the anticholinergic agent is a quaternary ammonium salt.

In its simplest form, the present invention involves a delivery system for the local application of an anticholinergic agent to the skin to prevent perspiration and thus indirectly enhance transdermal delivery of a pharmaceutically active drug. Local application may be first carried out with an anticholinergic agent in an amount sufficient to have a local antisecretory effect on the area treated, such amount being insufficient to cause a systemic effect, and then applying a transdermal delivery system to this area. Alternatively, the invention can be carried out by including the anticholinergic agent with the transdermal delivery system in such a manner that the anticholinergic agent can be delivered through the skin to prevent perspiration. The anticholinergic agent may be incorporated in a diffusion matrix, dispersed in a reservoir connected to such a matrix, included in a separate reservoir, dispersed in an ointment or connected in any other manner making it possible for the delivery of the anticholinergic agent through the skin to which the transdermal delivery system will be applied.

These and other objects of the invention will become apparent to those skilled in the art upon reading this disclosure.

## DETAILED DESCRIPTION OF THE INVENTION

There are a number of known chemical compounds which are known as "penetration enhancers". Such compounds interact in some manner with a pharmaceutically active drug and enhance the penetration of that drug through the skin. Perhaps the most famous of such penetration enhancers is "DMSO (dimethyl sulfoxide)". However, DMSO has not received FDA approval for use on humans. Another well known penetration enhancer is AZONE, see U.S. Patents 3,989,816, 4,311,481 and 4,316,893 as well as the corresponding foreign patents.

Such known penetration enhancers have a physical and/or chemical means of enhancing the penetration of the pharmaceutically active drug. Such compounds may themselves be easily transported through the skin and thus enhance the skin permeability, allowing transport of the pharmaceutically active drugs at significantly higher flux.

The enhancers may also have the effect of neutralizing molecular charges on the pharmaceutically active drugs, thus making them more susceptible to penetration of the skin.

Although such penetration enhancers are useful and might even be used in combination with the present invention, they do not cause the same physiological effect on the skin as does the present invention.

After making a thorough study of the transdermal delivery systems, the present inventor found that the transdermal delivery of pharmaceutically active drugs through the skin could be hindered by the outflow of perspiration from the skin and subsequent formation of an aqueous layer over the skin. The present inventor noted that transdermal delivery systems were normally worn for a long period of time, generally about twenty-four hours (24). When delivery systems are worn for such extended periods of time, substantial perspiration from the skin is very likely to build up between the outer layer of the skin and the contact layer of the transdermal delivery system. The aqueous layer of perspiration is thus formed between the delivery system and the skin interrupts the desired interface between the skin and the delivery system having an undesirable effect on the delivery of drugs from the system.

The present inventor noted that the above referred to problem could at times, be at least partially remedied by including additional pharmaceutically active drug into the delivery system. Such a solution is, of course, only possible where additional drug may be incorporated into the system without having undesirable effects. Such effects may take place where the pharmaceutically active drug acts as a solvent with respect to the system causing dissolution of the system. Further, even in situations where additional drug can be incorporated into the delivery system the problem is not completely solved in that individuals vary with respect to the amount they perspire and even a given individual may generate greatly different amounts of perspiration depending on the temperature, humidity and activity of the individual.

Due to individual differences in the amount of perspiration generated as well as the amount of perspiration a particular individual might be generating at any particular time, the present inventor found that the rate of drug delivery from a transdermal drug delivery system could vary. Such variance in the amount of drug delivered is, of course, undesirable in that the physician would prefer to prescribe a delivery system with which an accurate determination could be made with respect to the rate of delivery of the pharmaceutically active drug. The realization of the problems caused by perspiration when utilizing the transdermal delivery systems precipitated the discovery of the present invention by the present inventor.

After determining that perspiration from the skin causes problems with transdermal delivery systems, the present inventor determined that preventing that perspiration would solve the problems related thereto. According, an examination of various compounds utilized for preventing perspiration was made. Commercially available antiperspirants normally sold in the form of roll-ons and sprays are effective in preventing perspiration. Such compositions are normally aluminum based and prevent perspiration by a physical blocking means, i.e. the compounds actually clog the canals through which the perspiration passes. Since such compounds actually operate by clogging the passage ways out of the skin, the use of such compounds might not be desirable with respect to increasing the inflow of a pharmaceutically active drug through the skin. Although use of such compounds might avoid the formation of an aqueous layer between the skin and the transdermal delivery device, such compounds might themselves create barriers for the inflow of pharmaceutically active drugs through the skin.

The present inventor has done substantial work with anticholinergic compounds as shown in U.K. Patent 2,010,270 (incorporated herein by reference to disclose soft anticholinergic agents exhibiting antisecretory activity). Accordingly, the present inventor carried out experiments in order to determine if the use of such anticholinergic compounds would inhibit the inflow of pharmaceutically active drugs from a transdermal delivery device and found that the inflow of drugs was not inhibited. Knowing that such anticholinergic drugs prevented the outflow of perspiration and thus the formation of any aqueous layer, it was deduced that the effects of anticholingeric agents would increase the inflow of pharmaceutically active drugs through the skin.

Anticholinergic drugs affect the nervous system. The systemic effect of the nervous system is undesirable in connection with the present invention. Accordingly, the topical application of a small amount of anticholinergic agents to the skin is all that is necessary and desirable in order to obtain the results of the invention.

The normal working physiology of a human being provides a neurohumoral transmitter substance which provokes a stimulant action. For example, such a substance can evoke a stimulant action with respect to secretion glands within the skin and cause perspiration. However, within the normal physiology with a human, there is no comparable substance which provides an inhibitory action. There are, however, synthetic and plant-produced compounds that provide such inhibitory action. Such compounds act by blocking synaptic transmission in either the sympathetic or parasympathetic innervations. The present inventor noted that the blocking action of these inhibitors is usually quite specific to its locus. The use of such agents can cause blocking at the ganglionic nerve terminations (synapses) in either the sympathetic or parasympathetic ganglia, at the post ganglionic nerve terminations of either system (nerve-muscle junction blocking agent).

A chart is set forth below showing the various sites of blocking action of anticholinergic agents.

AUTONOMIC BLOCKING AGENTS

| SITE OF BLOCKING ACTION | NEUROHUMORAL TRANSMITTER SUBSTANCE | TYPE OF BLOCKING ACTION | EXAMPLE OF DRUG |
|---|---|---|---|
| Sympathetic Ganglion | Acetylcholine | Anticholinergic | Hexamethonium |
| Postganglionic Synapse | Norepinephrine (and epinephrine) | Antiadrenergic | Dibenamine |
| Parasympathetic Ganglion | Acetylcholine | Anticholinergic | Hexamethonium |
| Postganglionic Synapse | Acetylcholine | Anticholinergic | Atropine |
| Voluntary* Neuromuscular Junction | Acetylcholine | Anticholinergic | Curare |

*Included as a matter of convenience and because of certain similarities with the ganglionic blocking agents. These drugs are not autonomic blocking agents.

A number of different therapeutic actions can be obtained by the use of anticholinergic agents. The effect of such agents include the following:

1. Mydriatic effect (dilation of pupil of the eye) and cycloplegia (a paralysis of the ciliary structure of the eye, resulting in a paralysis of accommodation for near vision).
2. Antispasmodic effect (lowered tone and motility of the gastrointestinal tract and the genitourinary tract).
3. Antisecretory effect (reduced salivation (antisialogogue), reduced perspiration (anhydrotic) and reduced acid and gastric secretion.

As indicated above the anticholinergic agents have a substantial number of different effects on the human body. Accordingly, it is undesirable unless absolutely necessary to prescribe such agents in an amount such that they have a systemic effect. Therefore, it would be undesirable to prescribe such anticholinergic agents for oral usage simply to prevent perspiration which would then indirectly facilitate transdermal delivery of a drug from a transdermal delivery system. The mechanism of action of such drugs is obscure, but such drugs do affect a central mechanism. However, in order to affect a central mechanism and have a systemic effect the compounds must past the blood-brain barrier. Upon research, it was found that tertiary amines of such anticholinergic compounds can pass this blood-brain barrier but that quaternary ammonium compounds could not.

Tertiary amines of certain anticholinergic compounds have been shown to increase the brain acetylcholine levels in rats up to 40%. This increase coincides roughly with the onset of tremors similar to those observed in parkinsonism. The mechanism of acetylcholine increase in rats is uncertain but it has been shown not to be due to acetylcholinesterase inhibition or to activation to acetylase. However, the tremors are stopped effectively by administration of the tertiary amine type anticholinergic but not by the quaternary ammonium compounds.

Although any of the anticholinergic compounds can prevent secretion and thus aide in the transdermal delivery of a drug from a transdermal delivery system, due to the undesirable side effects of some of these compounds, the present inventor determined that it would be most desirable to utilize quaternary ammonium salts of known anticholinergic compounds, and has further determined that the topical application of such compounds in small amounts would be most desirable in terms of obtaining the desired effects of the present invention and avoiding undesirable side effects.

The following are specific examples of quaternary ammonium salts of anticholinergic compounds preferably used in connection with the present invention.

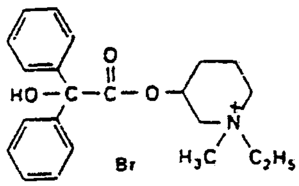

benzilonium Br (Portyn)

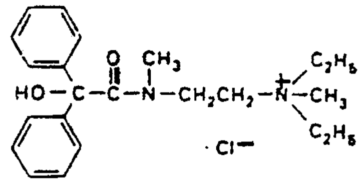

benzomethamine Cl (Contranul)

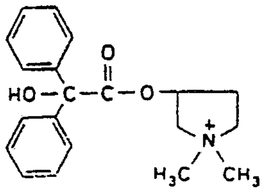

benzopyrrolate

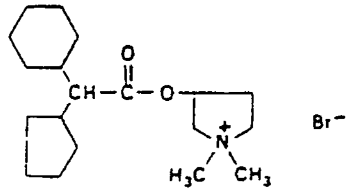

cyclopyrronium bromide

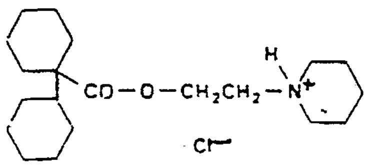

dihexyverine Cl (Metaspas)

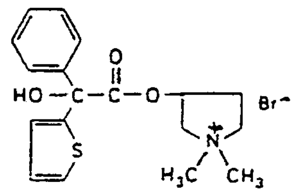

heteronium Br (Hetrum)

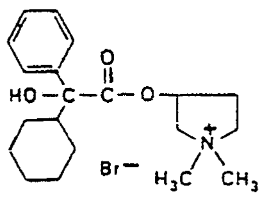

hexopyrronium Br

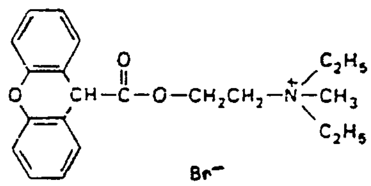

methantheline Br (Banthine)

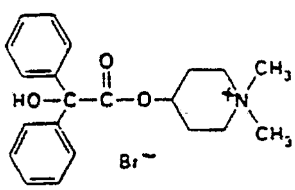

parapenzolate Br

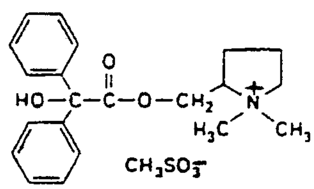

poldine mesylate (Nacton)

Glycopyrrolate methobromide (Robinul)

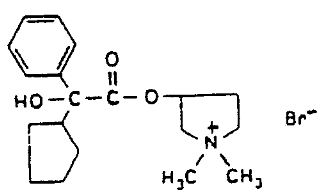

Mepenzolate methylbromide (Cantil)

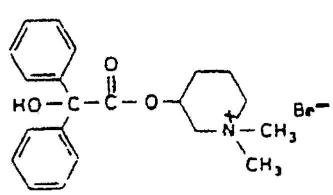

Oxyphenonium bromide
(Antrenyl)

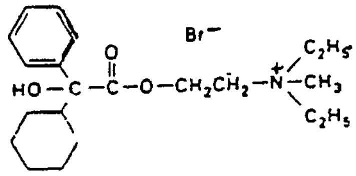

Penthienate bromide
(Monodral)

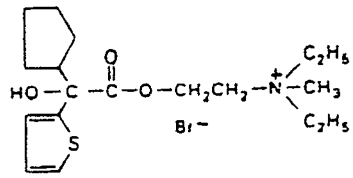

Pipenzolate methylbromide
(Piptal)

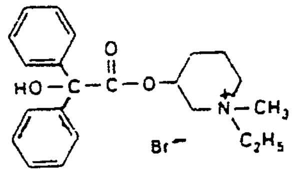

Propantheline bromide
(Pro-Banthine)

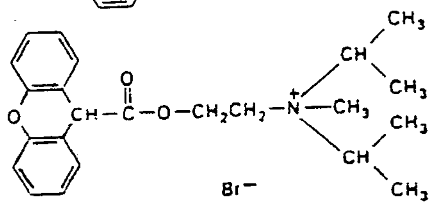

Diphemanil methylsulfate (Prantal)

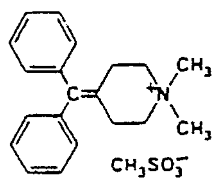

Hexocyclium methylsulfate (Tral)

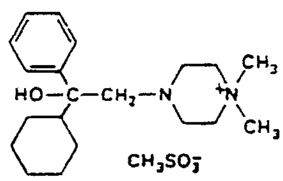

Thihexinol methylbromide (Entoquel)

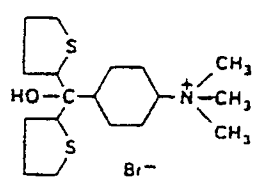

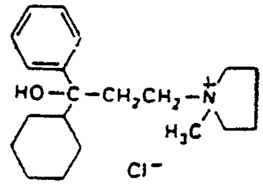

tricyclamol Cl (Elorine)

Anisotropine methylbromide (Valpin)

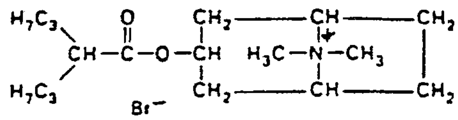

Homatropine methylbromide (Novatrin, and others)

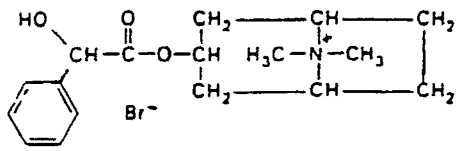

clidinium Br (Quarzan)

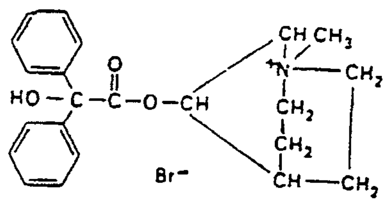

Methscopolamine bromide (Pamine, and others)

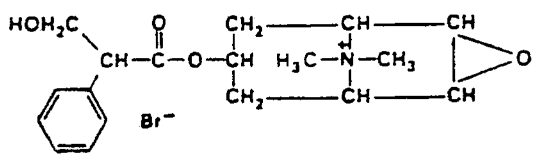

Tridihexethyl chloride (Pathilon)

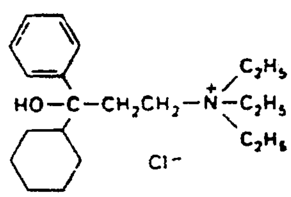

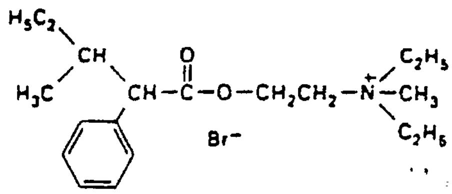

valethamate Br (Murel)

AMBUTONIUM BROMIDE

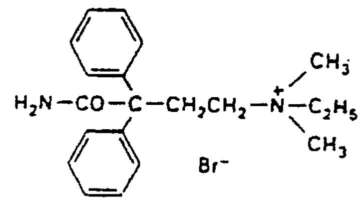

DIBUTOLINE SULFATE (Dibuline)

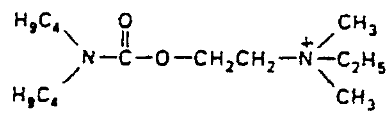

ISOPROPAMIDE IODIDE (Darbid)

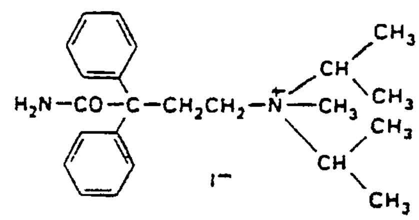

In order to describe some aspects of how the present invention works, the mechanism of a specific compound will be discussed. It is generally agreed that the activity of atropine-related anticholinergics is a competitive one with acetylcholine. Accordingly, the cholinomimetic agent (i.e. acetylcholine) possesses both affinity and intrinsic activity and can be bound to the receptor site and elicit the characteristic mimetic response. The anticholinergic agent, on the other hand, has the necessary affinity to bind firmly to the receptor but is unable to bring about an effective response, i.e., it has no intrinsic activity. The blocking agent, in sufficient concentration effectively competes for the receptor sites and prevents acetylcholine from binding thereon, thus preventing nerve activity.

With the present invention, the anticholingeric molecules have a primary point of attachment to cholinergic sites through the so called cationic head, i.e., the positively charged nitrogen present on the quaternary ammonium salts preferably used in connection with the present invention. Accordingly, the quaternary ammonium salts of anticholinergic agents of the present invention actually penetrate the skin and attach themselves on receptor sites. This prevents other agents which cause intrinsic activity from attaching to that site. When topically applied to the skin the anticholinergic agent physiologically interrupts the normal course of events and prevents perspiration. Unlike antiperspirants which are sold commercially, the anticholinergic agents of the present invention do not cause a physical blocking of any channels from which perspiration flows. The compounds of the present invention simply prevent secretion before it starts.

In order to determine if the anticholinergic agents of the present invention had any negative effect on the delivery of pharmaceutically active drugs from a transdermal delivery system, the inventor tested the invention on hairless mice. Knowing that hairless mice do not perspire, an anticholinergic agent was topically applied to an area of skin on hairless mice and a transdermal delivery system was then applied over the area to which the anticholinergic agent was applied. An identical transdermal delivery device was

applied to a different mouse to which no anticholinergic agent had been applied. The inventor found that both mice received the same amount of pharmaceutically active drug from the transdermal delivery system. Accordingly, it was deduced that the use of the anticholinergic agent had no effect on the transdermal delivery or the drug in an in vivo environment where no perspiration occurs. The present inventor then further deduced that the topical application of anticholinergic agents to human skin (where perspiration does occur) would facilitate the delivery of drugs from transdermal delivery systems in that such agents clearly do effectively prevent perspiration.

As indicated above, any type of anticholinergic agent can be utilized in connection with the present invention. However, the present inventor believes that quaternary ammonium compounds as indicated above and those disclosed within U.K. Patent 2,010,270 are preferably used in connection with the present invention.

The anticholinergic agents which are quaternary amonium salts as shown above and disclosed in U.K. 2,010,270 are believed to be more useful in connection with the present invention in that they are "soft" in nature, i.e., these agents exhibit substantial anticholinergic antisecretory activity, while having low toxicity following therapeutic application. They do not pass the "brain barrier." Other anticholinergic agents exhibit substantial anticholinergic antisecretory activity, but in addition these compounds give rise to a number of toxic side effects such as dizziness, blurred vision and dry mouth.

In order to obtain the effects of the delivery system of the present invention small amounts of the anticholinergic agents are utilized. For example, a useful composition can be prepared using anisotropine methylbromide (Valpin) as the anticholinergic agent (see Merck Index Tenth Edition, Entry Number 693). This agent is very soluble in alcohols. Accordingly, 20 milligrams can be dissolved in 25 milliliters of ethanol and 200 microliters of the solution can be applied to about five or six square centimeters of skin surface. This amount will prevent perspiration from that area for a period sufficient for the purposes of the present invention, i.e. about twenty-four (24) hours or more.

In order to make use of the present invention the anticholinergic agents can be dissolved in any suitable dermatologically acceptable solvent and applied by any known means to the surface area of skin to which a transdermal delivery device is to be applied. Alternatively, the agents may be included in any transdermal system. The anticholinergic agents of the invention are themselves capable of migrating transdermally. Accordingly, they can be merely placed on the skin, rubbed into the skin, sprayed on the skin or placed in contact with the skin as part of the transdermal delivery system. In order to obtain a cleansing effect which is an additional benefit when utilizing a transdermal delivery device, the anticholinergic agent can be placed onto an applicator such as a cotton swab or cloth which has the agent thereon dissolved in alcohol. The applicator having the agent dissolved thereon is then rubbed on the area of skin to which the device is to be applied. The alcohol has a cleansing antiseptic effect on the area and the anticholingeric compound is allowed to permeate the skin. The transdermal delivery device is then applied over the area which has been pre-treated. An agent known to enhance skin penetration might also be included on the applicator.

In addition to pre-treating an area of skin with an anticholinergic agent, it is possible to utilize the present invention by incorporating the anticholinergic agent into the transdermal delivery system. The penetration enhancers present in such a system might also enhance penetration of the anticholinergic agent. The agents of the invention can be incorporated into a matrix system, included into a reservoir with a pharmaceutically active drug, dispersed in an adhesive layer or ointment or otherwise included in connection with the system in some manner so that the agent may be delivered through the surface of the skin.

As indicated above, a large number of different types of pharmaceutically active drugs are now being considered for use in transdermal delivery systems. Further, as indicated above, a large number of different types of systems are utilized for delivering these drugs. Since the present invention can conceivably be used in connection with all types of transdermal delivery systems, the present inventor conceived of a preferred embodiment of his invention whereby the invention could be utilized in connection such systems without substantial modification

More specifically, anticholinergic agents of the invention could be absorbed on an applicator such as a piece of cotton cloth or other suitable soft pliable material, preferably in an alcohol type solvent. The solvent must be dermatologically acceptable and preferably have antiseptic properties. The applicator having the anticholinergic agent absorbed thereon is then incorporated into a separate packet or reservoir which is connected to the transdermal delivery system. This packet or reservoir can be opened prior to the application of the transdermal delivery system. Upon opening, the applicator is removed and the area of skin to which the system is to be applied is pre-treated by rubbing the area of skin with the applicator, thus cleansing the skin and allowing for transdermal delivery of the anticholinergic composition. After pre-treatment, the transdermal delivery device is applied to the pre-treated area. Delivery of the pharmaceutically active drug from the transdermal delivery system should be facilitated by the present invention in that perspiration from the pre-treated area of skin will be interrupted thus preventing the outflow of water from that area of skin and preventing the build up of any aqueous layer between the surface of the skin and the transdermal delivery system.

The present invention has been disclosed and described herein in what is believed to be its more preferred embodiments. However, upon reading this disclosure those skilled in the art will recognize modifications and variations thereof which are intended to be within the scope of the present invention.

## Claims

1. A transdermal delivery system capable of providing a desirable flow interface between the delivery system and skin surface comprising:
   a pharmaceutically active drug;
   a dermatologically and pharmacologically acceptable carrier means for the drug; and
   an anticholinergic agent in an amount sufficient to have a local antisecretory effect, such an amount being insufficient to cause a systemic effect.

2. A transdermal delivery system as claimed in Claim 1, wherein the anticholinergic agent is a quaternary ammonium salt.

3. A transdermal delivery system as claimed in Claim 1, wherein the carrier means is an absorption matrix positioned below a reservoir containing the pharmaceutically active drug and the anticholinergic agent is present in the reservoir with the pharmaceutically active drug.

4. A transdermal delivery system as claimed in Claim 1 , wherein the carrier means is a pressure-sensitive adhesive having the pharmaceutically active drug dispersed therein, the adhesive also having the anticholinergic agent dispersed therein.

5. A transdermal delivery system as claimed in Claim 1 , wherein the carrier means is a diffusion matrix having the pharmaceutically active drug dispersed therein along with the anticholinergic agent.

6. A transdermal delivery system as claimed in Claim 1 , further comprising:
   a separate reservoir containing the anticholinergic agent.

7. A transdermal delivery system as claimed in Claim 6 , wherein the anticholinergic agent is present in the separate reservoir in a liquid solution, the separate reservoir being easily openable for removal of the liquid solution therefrom.

8. A transdermal delivery system as claimed in Claim 6 , wherein the separate reservoir contains an applicator having absorbed thereon the anticholinergic agent, the applicator being easily removable from the reservoir to allow for application of the anticholinergic agent to the skin.

## Revendications

1. Système de libération transdermique capable de fournir une interface de flux souhaitable entre le système de libération et la surface de la peau comprenant :

   un médicament actif pharmaceutiquement;

   des moyens véhiculeurs pour le médicament, acceptables pharmaceutiquement et dermatologiquement; et

   un agent anti-cholinergique dans une quantité suffisante pour avoir un effet anti-secrétoire local, une telle quantité étant insuffisante pour entraîner un effet systémique.

2. Système de libération transdermique tel que revendiqué dans la revendication 1, où l'agent anti-cholinergique est un sel d'ammonium quaternaire.

3. Système de libération transdermique tel que revendiqué dans la revendication 1, où les moyens véhiculeurs sont une matrice d'absorption positionnée en-dessous d'un réservoir contenant le médicament actif pharmaceutiquement et où l'agent anti-cholinergique est présent dans le réservoir avec le médicament actif pharmaceutiquement.

4. Système de libération transdermique tel que revendiqué dans la revendication 1, où les moyens véhiculeurs sont un adhésif sensible à la pression à l'intérieur duquel sont dispersés le médicament actif pharmaceutiquement et également l'agent anti-cholinergique.

5. Système de libération transdermique tel que revendiqué dans la revendication 1, où les mcyens véhiculeurs sont une matrice de diffusion à l'intérieur de laquelle sont dispersés le médicament actif pharmaceutiquement ainsi que l'agent anti-cholinergique.

6. Système de libération transdermique tel que revendiqué dans la revendication 1, comprenant de plus :

un réservoir séparé contenant l'agent anti-cholinergique.

7. Système de libération transdermique tel que revendiqué dans la revendication 6, où l'agent anti-cholinergique est présent dans le réservoir séparé en une solution liquide, le réservoir séparé étant facilement ouvrable pour retirer de celui-ci la solution liquide.

8. Système de libération transdermique tel que revendiqué dans la revendication 6, où le réservoir séparé contient un applicateur sur lequel est absorbé l'agent anti-cholinergique, l'applicateur étant facilement retirable du réservoir pour permettre l'application de l'agent anti-cholinergique sur la peau.

## Ansprüche

1. Transdermales Abgabe-System, das zur Erzielung einer gewünschten Strömungs-Grenzfläche zwischen dem Abgabe-System und der Haut-Oberfläche befähigt ist, umfassend ein pharmazeutisch aktives Medikament,
ein dermatologisch und pharmakologisch annehmbares Träger-Mittel für das Medikament und
ein anticholinergisches Mittel in einer Menge, die zur Ausübung eines lokalen antisekretorischen Effekts ausreicht, wobei eine derartige Menge nicht ausreicht, um einen systemischen Effekt zu verursachen.

2. Transdermales Abgabe-System nach Anspruch 1, worin das anticholinergische Mittel ein quaternäres Ammonium-Salz ist.

3. Transdermales Abgabe-System nach Anspruch 1, worin das Träger-Mittel eine Absorptions-Matrix ist, die unterhalb eines das pharmazeutisch aktive Medikament enthaltenden Vorratsbehälters angeordnet ist, und das anticholinergische Mittel in dem Vorratsbehälter mit dem pharmazeutisch aktiven Medikament anwesend ist.

4. Transdermales Abgabe-System nach Anspruch 1, worin das Träger-Mittel ein druckempfindliches Haftmittel ist, das das pharmazeutisch aktive Medikament darin dispergiert aufweist, wobei das Haftmittel auch das anticholinergische Mittel darin dispergiert aufweist.

5. Transdermales Abgabe-System nach Anspruch 1, worin das Träger-Mittel eine Diffusions-Matrix ist, die das pharmazeutisch aktive Medikament zusammen mit dem anticholinergischen Mittel darin dispergiert aufweist.

6. Transdermales Abgabe-System nach Anspruch 1, weiterhin umfassend
einen getrennten Vorratsbehälter, der das anticholinergische Mittel enthält.

7. Transdermales Abgabe-System nach Anspruch 6, worin das anticholinergische Mittel in einem getrennten Vorratsbehälter in einer flüssigen Lösung vorliegt, wobei der getrennte Vorratsbehälter zur Entfernung der flüssigen Lösung aus demselben leicht zu öffnen ist.

8. Transdermales Abgabe-System nach Anspruch 6, worin der getrennte Vorratsbehälter einen Applikator enthält, der das anticholinergische Mittel darauf absorbiert enthält, wobei der Applikator sich leicht von dem Vorratsbehälter abnehmen läßt, um das Auftragen des anticholinergischen Mittels auf die Haut zu ermöglichen.